# EUROPEAN PATENT APPLICATION

(11) **EP 2 364 680 A1**
(43) Date of publication of application: **14.09.2011**
(21) Application number: 11164915.8
(22) Date of filing: 22.02.2006
(51) Int. Cl.: A61F 9/00, A61K 9/00

(54) **Microimplants for ocular administration**

(30) Priority: 01.03.2005 US 70158
(62) Divisional of application: 06735790.5
(71) Applicant: Allergan, Inc., Irvine, CA 92612 (US)
(72) Inventor: The designation of the inventor has not yet been filed
(74) Representative: HOFFMANN EITLE

(57) **Abstract**

Implants and microimplants are provided comprising homogeneous mixtures of active ingredient and bioerodible polymer, including microimplant having diameters of (0.019) inches or less and which can be delivered to the eye using self-sealing methods. Methods of manufacturing the implants and microimplants are provided that yield batches of highly uniform implants and microimplants within vary narrow tolerance ranges.

## Description

The present invention relates to implants that include active ingredients, such as drugs or other therapeutic agents. More particularly, the present invention relates to implants that can be inserted into the eye to treat ocular diseases or conditions.

A primary difficulty in treating diseases or conditions of the eye is introducing active ingredients, such as drugs or other therapeutic agents into the eye and maintaining these active ingredients at a therapeutically effective concentration in the eye for the necessary duration. Systemic administration may not be an ideal solution because, often, unacceptably high levels of systemic dosing is needed to achieve effective intraocular concentrations, with the increased incidence of unacceptable side effects of the active ingredients. Simple ocular instillation or application is not an acceptable alternative in many cases because the active ingredients can be quickly washed out by tear-action or can be depleted from within the eye into the general circulation. Suprachoroidal injections of drug solutions have also been performed, but again drug availability is short-lived. It remains difficult to maintain therepeutic levels of drug for adequate time periods without repeated dosing.

Efforts to address this problem have led to the development of implants that can be inserted into the eye such that a controlled amount of a desired active ingredient can be released constantly over a period of several days, weeks, or even months. Many such devices have been previously reported. See, for example, U.S. Patent No. 4,853,224, which discloses biocompatible implants for introduction into the anterior or posterior segment of an eye for the treatment of an ocular condition. U.S. Patent No. 5,164,188 discloses a method of treating an ocular condition by introducing a biodegradable implant comprising drugs of interest into the suprachoroidal space or pars plana of the eye. See also U.S. Patent Nos. 5,824,072; 5,476,511; 4,997,652; 4,959,217; 4,668,506; and 4,144,317. Other methods include anchoring a plug or tack containing a drug into the sclera of the eye (see, e.g., U.S. Pat. No. 5,466,233).

Various sites exist in the eye for insertion of an implant, such as the vitreous of the eye, anterior or posterior chambers of the eye, or other areas of the eye including intraretinal, subretinal, intrachoroidal, suprachoroidal, intrascleral, episcleral, subconjunctival, intracorneal, or epicorneal spaces. Wherever the desired location of insertion, typical methods of insertion all require relatively invasive surgical procedures, pose a risk of excessive trauma to the eye, and require excessive handling of the implant. For example, in a typical method for placement into the vitreous, an incision is made through the sclera, and the implant is inserted into the vitreous, using forceps or other like manual grasping device. Once inserted, the forceps (or grasping device) is removed, and the incision is sutured closed. Alternatively, an incision can be made through the sclera, a trocar can be advanced through the incision, and the implant can be delivered through the trocar. Similar methods can be employed to deliver implants to other locations, e.g., insertion in the anterior chamber of the eye through an incision in the cornea.

The drawbacks of such techniques are many-fold. Extensive handling of the implant is necessitated in these techniques, creating a risk that the implant will be damaged in the process. Many such implants are polymer-based and are relatively fragile. If portions of such implants are damaged or broken-off, the effective therapeutic dose delivered by the implant once placed will be significantly altered. In addition, it becomes inherently difficult using these methods to achieve reproducible placement from patient to patient. Also of import is the fact that all such techniques require an incision or puncture in the eye large enough to require suturing. Thus, such techniques are typically performed in a surgical setting.

Accordingly, minimally invasive methods of introducing implants into the eye which minimize or even eliminate the need for surgery are desirable, including methods which would introduce implants small enough to allow the eye to self-seal after insertion, without the need for suturing. An important feature in such minimally invasive methods would be the use of smallest possible implant, which in turn would result in less trauma to the eye upon implantation. However, such small implants are difficult to manufacture, in particular, because these small implants must have homogenous compositions and must have consistent shapes, sizes, and morphologies to implement the desired dosage levels of active ingredients over a desired period of time. Even slight variations in the homogeneity of the compositions or consistency in the shapes, sizes, or morphologies of these small implants can result in the delivery of a drastically different amount of active ingredient than expected, and can alter the treatment of an ocular disease or condition. Accordingly, producing small implants having uniform compositions, shapes, sizes, and morphologies, and in particular, producing them on a commercially feasible scale, is of considerable interest.

### SUMMARY

The present invention meets these needs and provides other advantages. In certain aspects of the invention, microimplants are provided that comprise homogeneous mixtures of active ingredient, such as one or more therapeutic agents, and a bioerodible polymer or polymers and which have diameters of 0.020 inches or less, preferably 0.019 inches or less and most preferably 0.018 inches or less, Preferably, these diameters are within ±0.0003 inches of a mean diameter, such as the diameters identified above. In other aspects of the present invention, microimplant as well as larger implants can be manufactured in batches that meet rigid specifications, including specified mass tolerances of less than 10% by weight or less of the desired target mass. Similarly, such implants or microimplants can be manufactured in batches having a mass standard deviation of about 5% or less. In one aspect of the invention, microimplant are provided that include an anti-inflammatory agent, such as dexamethasone, and the bioerodible polymers used are based upon polylactic acid polyglycolic acid (PLGA) copolymers. Such microimplants are about 7 millimeters in length or less and can have diameters of 0.019 inches or less. In one variation, such microimplants further include the addition of a polylactic acid polyglycolic acid (PLGA) copolymer having free acid end groups. The invention further provides for batches or populations of such microimplant within a specified weight percentage of the desired target mass, and/or having specified mass standard deviations.

In other aspects of the invention, methods are provided to produce implants and microimplants within very narrow tolerances for variations in composition and/or dimensions. Such methods include steps of obtaining particles or powders of one or more active ingredients, polymers, and/or other optional excipients (e.g., release modulators, buffers, etc.), blending these particles into a mixture, extruding this mixture into filaments, pelletizing and extruding filaments using a twin screw extruder to obtain a high degree of homogenity, and precisely cutting these filaments into desired microimplants. Single and dual extrusion methods can be used.

In yet other aspects of the invention, methods and criteria are provided for accepting or rejecting batches of microimplants based on optical, mass, dimensional or other measurements.

Each and every feature described herein, and each and every combination of two or more of such features, is included within the scope of the present invention provided that the features included in such a combination are not mutually inconsistent. In addition, any feature or combination of features may be specifically excluded from any embodiment of the present invention.

Additional aspects and advantages of the present invention are set forth in the following description and claims, particularly when considered in conjunction with the accompanying drawings.

### DESCRIPTION

In order to provide a more thorough understanding of the present invention, the following description sets forth numerous specific details, such as specific configurations, parameters, or the like. It should be recognized, however, that such description is not intended as a limitation on the scope of the present invention, but is intended to provide a better description of the exemplary embodiments.

Implants and microimplants prepared according to the present invention can be inserted into the eye to treat ocular diseases and conditions. Such implants or microimplants will typically include compositions of active ingredient(s) and bioerodible polymer(s), with the optional inclusion of additional excipient.

As used herein, the term "implant" is meant to include any ocular implant or drug delivery device that can be inserted into any number of locations in the eye and that can release a controlled amount of active ingredient over a sustained period of time, including days, weeks, or even months. Such implants are biocompatible, and in many but not all cases are formed of a bioerodible substance, such as a bioerodible polymer. "Microimplants" refers to implants having a sufficiently small cross-sectional area that they can be delivered according to methods that result in self-sealing of the eye at the puncture site associated with the delivery. In particular, such microimplants have dimensions such that they are deliverable through 20 gauge, 21 gauge or 22 gauge or smaller sized cannulas. Thin wall versions of 21 gauge needles can be manufactured having inner diameters of up to 0.028 inches, thus cylindrical microimplants deliverable through such cannulas will have outer diameters of less than 0.028 inches. Microimplants can also have non-circular cross-sectional geometries for delivery through cannulas having corresponding cross-sectional geometries. Where the micro-implant has non-circular cross-section, the cross-sectional area may be up to 0.00025 square inches or more, depending on the particular cross-sectional geometry. Implants, including microimplants, may be understood to comprise relatively smaller units of one or more active ingredients. For example, an implant may comprise a population of particles of an active ingredient. As used herein, particles may have any shape and are smaller in size than an implant that is administered to a patient to treat an ocular condition. In contrast to liquid ophthalmic compositions, the present implants are substantially solid, at least initially, before administration to a patient in need of ocular therapy.

As used herein, "active ingredient" means any pharmacologically active agent, either alone or in combination, for which sustained or controlled release is desirable and may be employed, including but not limited to the agents listed herein.

The term "bioerodible polymer" refers to a polymer or polymers which degrade is vivo, and wherein erosion of the polymer over time is required to achieve desired agent release kinetics.

Microimplants prepared according to the present invention can be sufficiently small, or miniaturized, in size and shape such that they can be inserted into the eye without the necessity of an incision or puncture wound made in the eye that would normally require suturing or other surgical procedure to repair, as is typically the case when implanting larger implants. With the present microimplants, and according to insertion techniques further described below, the eye can "self-seal" after insertion of the microimplant, thereby eliminating the need for suturing or surgery, and the pain and trauma associated therewith, and also avoiding the costs, time and other incoveniences of performing such procedures in a surgical setting. As used herein, "self sealing" methods of delivering microimplants into the eye refers to methods of introducing one or more microimplants through a cannula and into desired locations of a patient's eye without the need for a suture, or other like closure means, at the cannula puncture site. Such "self sealing" methods do not require that the puncture site completely seal immediately upon withdrawal of the cannula, but rather that any initial leakage is minimal and dissipates in short order such that a surgeon or another person skilled in the art, in his or her good clinical judgment, would not be compelled to suture or otherwise provide other like closure means to the puncture site.

Both larger implants and smaller microimplants produced according to the present invention will have highly uniform characteristics and thus retain the ability to deliver precise and accurate dosage of the active ingredient, thereby providing for a highly controlled rate of release of the active ingredient into the eye over a specified time. The active ingredient released from the present implants may selectively target certain regions of the eye. For example, for implants placed in the posterior segment of an eye of a patient, the active ingredient may be released from the implant so that the active ingredient provides a therapeutic benefit to the retina, or a portion of the retina of the eye in which the implant is placed

The present implants can be produced by combining particles of an active ingredient or ingredients and particles of a bioerodible polymer or polymers. According to particular processes, microimplants can be produced trough a manufacturing process that includes sorting particles made of an active ingredient or ingredients and particles made of a bioerodible polymer or polymers according to size, blending these particles into a homogenous mixture of active ingredient(s) and bioerodible polymer(s), extruding (using a single or dual extrusion method) this mixture into filaments, precisely cutting these filaments into microimplants, and inspecting these microimplants for desired characteristics. This manufacturing process can be used to produce batches or populations of microimplants having the desired uniform characteristics, such as weight, dimensions (e.g., length, diameter, area, volume), regional distribution of active ingredients, release kinetics, and the like. For instance, these batches can be subjected to a specified acceptance critieria, including, e.g., a specified mass criteria, such as within a certain weight percentage of the desired target weight per microimplant, or e.g., a specified acceptable mass standard deviation for the microimplants of each batch. A specific drug/polymer ratio can also be obtained so that each individual implant of a batch or population of implants can contain the same amount of active ingredient. Thus, a batch of implants, such as a batch of implants in a package, may be provided with a specific label strength or dose of active ingredient for each individual implants of that batch.

### 1. Compositions of Implants and Microimplants

Generally, implants and microimplants produced according to the invention can be formulated with a mixture of an active ingredient or ingredients and a bioerodible polymer or polymers, which can together control the release kinetics of the active ingredient into the eye. The particular formulation may vary according to the preferred drug release profile, the particular active ingredient or ingredients being used, the site of implantation, the condition being treated, and the medical history of the patient, for example. The present implants are formulated with particles of the active ingredient entrapped within the bioerodible polymer matrix. Release of the active ingredient or ingredients is achieved by erosion of the polymer followed by exposure of previously entrapped or dispersed active ingredient particles to the eye, and subsequent dissolution and release of agent. The parameters which determine the release kinetics include, but are not limited to, the size of the drug particles, the water solubility of the drug, the ratio of drug to polymer, the particular methods of manufacture, the shape of the implant, the surface area exposed, and the erosion rate of the polymer.

### Active ingredients

Various pharmacologically active agents, therapeutics, and drugs of interest can be provided in the present implants. Non-limiting examples of pharmacologically active agents include anti-glaucoma drugs, such as the beta-blockers: timolol maleate, betaxolol and metipranolol; mitotics: pilocarpine, acetylcholine chloride, isoflurophate, demacarium bromide, echothiophate iodide, phospholine iodide, carbachol, and physostigimine; epinephrine and salts, such as dipivefrin hydrochloride; and dichlorphenamide, acetazolamide and methazolamide; anti-cataract and anti-diabetic retinopathy drugs, such as aldose reductase inhibitors: tolrestat, lisinopril, enalapril, and statil; thiol crosslinking drugs other than those considered previously; anti-cancer drugs, such as retinoic acid, methotrexate, adriamycin, bleomycin, triamcinolone, mitomycin, cis-platinum, vincristine, vinblastine, actinomycin-D, ara-c, bisantrene, CCNU, activated cytoxan, DTIC, HMM, melphalan, mithramycin, procarbazine, VM26, VP16, and tamoxifen; immune modulators, other than those indicated previously; anti-clotting agents, such as tissue plasminogen activator, urokinase, and streptokinase; anti-tissue damage agents, such as superoxide dismutase; proteins and nucleic acids, such as mono- and polyclonal antibodies, enyzmes, protein hormones and genes, gene fragments and plasmids; steroids, particularly steroidal anti-inflammatory agents or anti-fibrous drugs, such as cortisone, hydrocortisone, prednisolone, prednisone, dexamethasone, progesterone-like compounds, medrysone (HMS) and fluorometholone; non-steroidal anti-inflammatory drugs, such as ketrolac tromethamine, diclofenac sodium and suprofen; antibiotics, such as loridine (cephaloridine), chloramphenicol, clindamycin, amikacin, tobramycin, methicillin, lincomycin, oxycillin, penicillin, amphotericin B, polymyxin B, cephalosporin family, ampicillin, bacitracin, carbenicillin, cepholothin, colistin, erythromycin, streptomycin, neomycin, sulfacetamide, vancomycin, silver nitrate, sulfisoxazole diolamine, and tetracycline; other anti-pathogens, including anti-viral agents, such as idoxoridine, trifluorouridine, vidarabine (adenine arabinoside), acyclovir (acycloguanosine), gancyclovir, pyrimethamine, trisulfapyrimidine-2, clindamycin, nystatin, flucytosine, natamycin, miconazole and piperazine derivatives, e.g. diethylcarbamazine; cycloplegic and mydriatic agents, such as atropine, cyclogel, scopolamine, homatropine and mydriacyl.

Other agents include anticholinergics, anticoagulants, antifibrinolytic agents, antihistamines, antimalarials, antitoxins, chelating agents, hormones, immunosuppressives, thrombolytic agents, vitamins, salts, desensitizing agents, prostaglandins, amino acids, metabolites and antiallergenics.

Some active ingredients may be provided in the present implants with release modulators. Certain therapeutically active hydrophobic agents which may further benefit from release modulators include cyclosporines, e.g. cyclosporin A, cyclosporin G, etc.; vinca alkaloids, e.g. vincristine and vinblastine; methotrexate; retinoic acid; certain antibiotics, e.g. ansamycins such as rifampin; nitrofurans such as nifuroxazide; non-steroidal antiinflammatory drugs, e.g. diclofenac, keterolac, flurbiprofen, naproxen, suprofen, ibuprofen, aspirin, etc. Steroids are of interest, including those previously mentioned as well as others, e.g., estrogens, progesterones, and the like.

The present implants may also include a therapeutic combination of two or more active agents, which provides for a sustained release of the agents. Combinations may include steroids, as indicated above, as the hydrophobic agent and water soluble antibiotics, e.g. aminoglycosides such as gentamycin, kanamycin, neomycin, and vancomycin; amphenicols such as chloramphenicol; cephalosporins, such as cefazolin HCl; penicillins such as ampicillin, penicillin, carbenicillin, oxycillin, methicillin; lincosamides such as lincomycin; polypeptide antibiotics such as polymixin and bacitracin; tetracyclines such as tetracycline; quinolones such as ciproflaxin, and the like; sulfonamides such as chloramine T; and sulfones such as sulfanilic acid as the hydrophilic entity. A combination of non-steroidal anti-inflammatory drugs, as indicated above, with water soluble antibiotics is also of interest. Combinations of anti-viral drugs, e.g. acyclovir, gancyclovir, vidarabine, azidothymidine, dideoxyinosine, dideoxycytosine with steroidal or non-steroidal anti-inflammatory drugs, as indicated above, are of interest.

A combined anti-inflammatory drug, and antibiotic or antiviral, may be further combined with an additional therapeutic agent. The additional agent, which may also be used independently or in other combinations, may be an analgesic, e.g. codeine, morphine, keterolac, naproxen, and the like, an anesthetic, e.g. lidocaine; β-adrenergic blocker or β-adrenergic agonist, e.g. ephidrine, epinephrine, and the like; aldose reductase inhibitor, e.g. epalrestat, ponalrestat, sorbinil, tolrestat; antiallergic, e.g. cromolyn, beclomethasone, dexamethasone, and flunisolide; colchicine. Anihelminthic agents, e.g. ivermectin and suramin sodium; antiamebic agents, e.g. chloroquine and chlortetracycline; and antifungal agents, e.g. amphotericin, and the like may be used independently or co-formulated with an antibiotic and/or an anti-inflammatory drug. For intra-ocular use, anti-glaucomas agents, e. g. acetozolamide, befunolol, and the like, alone or in combinations with anti-inflammatory and antimicrobial agents are of interest. For the treatment of neoplasia, anti-neoplastics or combinations with anti-neoplastics, particularly vinblastine, vincristine, interferons α, β and γ, antimetabolites, e.g. folic acid analogs, purine analogs, pyrimidine analogs may be used. Immunosuppressants such as azathiprine, cyclosporine and mizoribine are of interest alone in combinations. Also useful alone or in combinations include miotic agents, e.g. carbachol, mydriatic agents such as atropine, and the like, protease inhibitors such as aprotinin, camostat, gabexate, vasodilators such as bradykinin, and the like, and various growth factors, such epidermal growth factor, basic fibroblast growth factor, nerve growth factors, and the like.

Additional therapeutic agents, such as antimetabolites and/or antibiotics, are useful alone or in combination. Antimetabolites include, but are not limited to, folic acid analogs (*e.g.*, denopterin, edatrexate, methotrexate, piritrexim, pteropterin, Tomudex®, trimetrexate), purine analogs (*e.g.*, cladribine, fludarabine, 6-mercaptopurine, thiamiprine, thiaguanine), and pyrimidine analogs (*e.g.*, ancitabine, azacitidine, 6-azauridine, carmofur, cytarabine, doxifluridine, emitefur, enocitabine, floxuridine, fluorouracil, gemcitabine, tegafur).

For steroidal anti-inflammatory agents, prefereably the agent is selected from the group consisting of 21-acetoxypregnenolone, alclometasone, algestone, amcinonide, beclomethasone, betamethasone, budesonide, chloroprednisone, clobetasol, clobetasone, clocortolone, cloprednol, corticosterone, cortisone, cortivazol, deflazacort, desonide, desoximetasone, dexamethasone, diflorasone, diflucortolone, difluprednate, enoxolone, fluazacort, flucloronide, flumethasone, flunisolide, fluocinolone acetonide, fluocinonide, fluocortin butyl, fluocortolone, fluorometholone, fluperolone acetate, fluprednidene acetate, fluprednisolone, flurandrenolide, fluticasone propionate, formocortal, halcinonide, halobetasol propionate, halometasone, halopredone acetate, hydrocortamate, hydrocortisone, Ioteprednol etabonate, mazipredone, medrysone, meprednisone, methylprednisolone, mometasone furoate, paramethasone, prednicarbate, prednisolone, prednisolone 25-diethylamino-acetate, prednisolone sodium phosphate, prednisone, prednival, prednylidene, rimexolone, tixocortol, triamcinolone, triamcinolone acetonide, triamcinolone benetonide, and triamcinolone hexacetonide. In a preferred embodiment, the steroidal anti-inflammatory agent is selected from the group consisting of cortisone, dexamethasone, hydrocortisone, methylprednisolone, prednisolone, prednisone, and triamcinolone. In a more preferred embodiment, the steroidal anti-inflammatory agent is dexamethasone. In another embodiment, the bioerodible implant comprises more than one steroidal anti-inflammatory agent.

Specific antibiotics that can be used include, but are not limited to:
Antibacterial antibiotics, such as aminoglycosides (*e.g*., amikacin, apramycin, arbekacin, bambermycins, butirosin, dibekacin, dihydrostreptomycin, fortimicin(s), gentamicin, isepamicin, kanamycin, micronomicin, neomycin, neomycin undecylenate, netilmicin, paromomycin, ribostamycin, sisomicin, spectinomycin, streptomycin, tobramycin, trospectomycin), amphenicols (*e.g*., azidamfenicol, chloramphenicol, florfenicol, thiamphenicol), ansamycins (*e.g*., rifamide, rifampin, rifamycin sv, rifapentine, rifaximin), b-lactams (*e.g*., carbacephems (*e.g*., loracarbef), carbapenems (*e.g*., biapenem, imipenem, meropenem, panipenem), cephalosporins (*e.g*., cefaclor, cefadroxil, cefamandole, cefatrizine, cefazedone, cefazolin, cefcapene pivoxil, cefclidin, cefdinir, cefditoren, cefepime, cefetamet, cefixime, cefmenoxime, cefodizime, cefonicid, cefoperazone, ceforanide, cefotaxime, cefotiam, cefozopran, cefpimizole, cefpiramide, cefpirome, cefpodoxime proxetil, cefprozil, cefroxadine, cefsulodin, ceftazidime, cefteram, ceftezole, ceftibuten, ceftizoxime, ceftriaxone, cefuroxime, cefuzonam, cephacetrile sodium, cephalexin, cephaloglycin, cephaloridine, cephalosporin, cephalothin, cephapirin sodium, cephradine, pivcefalexin), cephamycins (*e.g*., cefbuperazone, cefmetazole, cefminox, cefotetan, cefoxitin), monobactams (*e.g*., aztreonam, carumonam, tigemonam), oxacephems, flomoxef, moxalactam), penicillins (*e.g*., amdinocillin, amdinocillin pivoxil, amoxicillin, ampicillin, apalcillin, aspoxicillin, azidocillin, azlocillin, bacampicillin, benzylpenicillinic acid, benzylpenicillin sodium, carbenicillin, carindacillin, clometocillin, cloxacillin, cyclacillin, draloxacillin, epicillin, fenbenicillin, floxacillin, hetacillin, lenampicillin, metampicillin, methicillin sodium, mezlocillin, nafcillin sodium, oxacillin, penamecillin, penethamate hydriodide, penicillin g benethamine, penicillin g benzathine, penicillin g benzhydrylamine, penicillin g calcium, penicillin g hydrabamine, penicillin g potassium, penicillin g procaine, penicillin n, penicillin o, penicillin v, penicillin v benzathine, penicillin v hydrabamine, penimepicycline, phenethicillin potassium, piperacillin, pivampicillin, propicillin, quinacillin, sulbenicillin, sultamicillin, talampicillin, temocillin, ticarcillin), other (*e.g*., ritipenem), lincosamides (*e.g*., clindamycin, lincomycin), macrolides (*e.g*., azithromycin, carbomycin, clarithromycin, dirithromycin, erythromycin, erythromycin acistrate, erythromycin estolate, erythromycin glucoheptonate, erythromycin lactobionate, erythromycin propionate, erythromycin stearate, josamycin, leucomycins, midecamycins, miokamycin, oleandomycin, primycin, rokitamycin, rosaramicin, roxithromycin, spiramycin, troleandomycin), polypeptides (*e.g*., amphomycin, bacitracin, capreomycin, colistin, enduracidin, enviomycin, fusafungine, gramicidin s, gramicidin(s), mikamycin, polymyxin, pristinamycin, ristocetin, teicoplanin, thiostrepton, tuberactinomycin, tyrocidine, tyrothricin, vancomycin, viomycin, virginiamycin, zinc bacitracin), tetracyclines (*e.g*., apicycline, chlortetracycline, clomocyctine, demeclocycline, doxycycline, guamecycline, lymecycline, meclocycline, methacycline, minocycline, oxytetracycline, penimepicycline, pipacycline, rolitetracycline, sancycline, tetracycline), and others (*e.g*., cycloserine, mupirocin, tuberin).

Synthetic antibacterials, such as 2,4-Diaminopyrimidines (*e.g*., brodimoprim, tetroxoprim, trimethoprim), nitrofurans (*e.g*., furaltadone, furazolium chloride, nifuradene, nifuratel, nifurfoline, nifurpirinol, nifurprazine, nifurtoinol, nitrofurantoin), quinolones and analogs (*e.g*., cinoxacin, ciprofloxacin, clinafloxacin, difloxacin, enoxacin, fleroxacin, flumequine, grepafloxacin, lomefloxacin, miloxacin, nadifloxacin, nalidixic acid, norfloxacin, ofloxacin, oxolinic acid, pazufloxacin, pefloxacin, pipemidic acid, piromidic acid, rosoxacin, rufloxacin, sparfloxacin, temafloxacin, tosufloxacin, trovafloxacin), sulfonamides (*e.g*., acetyl sulfamethoxypyrazine, benzylsulfamide, chloramine-b, chloramine-t, dichlorsmine t, n²-formylsulfisomidine, n⁴-b-d-glucosylsulfanilamide, mafenide, 4'-(methylsulfamoyl)sulfanilanilide, noprylsulfamide, phthalylsulfacetamide, phthalylsulfathiazole, salazosulfadimidine, succinylsulfathiazole, sulfabenzamide, sulfacetamide, sulfachlorpyridazine, sulfachrysoidine, sulfacytine, sulfadiazine, sulfadicramide, sulfadimethoxine, sulfadoxine, sulfaethidole, sulfaguanidine, sulfaguanol, sulfalene, sulfaloxic acid, sulfamerazine, sulfameter, sulfamethazine, sulfamethizole, sulfamethomidine, sulfamethoxazole, sulfamethaxypyridazine, sulfametrole, sulfamidochrysoidine, sulfamoxole, sulfanilamide, 4-sulfanilamidosalicylic acid, n⁴-sulfanilylsulfanilamide, sulfanilylurea, n-sulfanilyl-3,4-xylamide, sulfanitran, sulfaperine, sulfaphenazole, sulfaproxyline, sulfapyrazine, sulfapyridine, sulfasomizole, sulfasymazine, sulfathiazole, sulfathiourea, sulfatolamide, sulfisomidine, sulfisoxazole) sulfones (*e.g*., acedapsone, acediasulfone, acetosulfone sodium, dapsone, diathymosulfone, glucosulfone sodium, solasulfone, succisulfone, sulfanilic acid, p-sulfanilylbenzylamine, sulfoxone sodium, thiazolsulfone), and others (*e.g*., clofoctol, hexedine, methenamine, methenamine anhydromethylene-citrate, methenamine hippurate, methenamine mandalate, methenamine sulfosalicylate, nitroxline, taurolidine, xibornol),

Antifungal antibiotics, such as polyenes (*e.g*., amphotericin b, candicidin, dermostatin, filipin, fungichromin, hachimycin, hamycin, lucensomycin, mepartricin, natamycin, nystatin, pecilocin, perimycin), others (*e.g*., azaserine, griseofulvin, oligomycins, neomycin, undecylenate, pyrrolnitrin, siccanin, tubercidin, viridin).

Synthetic antifungals such as allylamines (*e.g*., butenafine, naftifine, terbinafine), imidazoles (*e.g*., bifonazole, butoconazole, chlordantoin, chlormidazole, cloconazole, clotrimazole, econazole, enilconazole, fenticonazole, flutrimazole, isoconazole, ketoconazole, lanoconazole, miconazole, omoconazole, oxiconazole nitrate, sertaconazole, sulconazole, tioconazole), thiocarbamates (*e.g*., tolciclate, tolindate, tolnaftate), triazoles (*e.g*., fluconazole, itraconazole, saperconazole, terconazole) others (*e.g*., acrisorcin, amorolfine, biphenamine, bromosalicylchloranilide, buclosamide, calcium propionate, chlorphenesin, ciclopirox, cloxyquin, coparaffinate, diamthazole dihydrochloride, exalamide, flucytosine, halethazole, hexetidine, loflucarban, nifuratel, potassium iodide, propionic acid, pyrithione, salicylanilide, sodium propionate, sulbentine, tenonitrozole, triacetin, ujothion, undecylenic acid, zinc propionate).

Other antibiotics and analogs (*e.g*., aclacinomycins, actinomycin f₁, anthramycin, azaserine, bleomycins, cactinomycin, carubicin, carzinophilin, chromomycins, dactinomycin, daunorubicin, 6-diaxo-5-oxo-L-norleucine, doxorubicin, epirubicin, idarubicin, menogaril, mitomycins, mycophenolic acid, nogalamycin, olivomycines, peplomycin, pirarubicin, plicamycin, porfiromycin, puromycin, streptonigrin, streptozocin, tubercidin, zinostatin, zorubicin), antimetabolites (*e.g*. folic acid analogs (*e.g*., denopterin, edatrexate, methotrexate, piritrexim, pteropterin, Tomudex^{®}, trimetrexate), purine analogs (*e.g*., cladribine, fludarabine, 6-mercaptopurine, thiamiprine, thioguanine), pyrimidine analogs (*e.g*., ancitabine, azacitidine, 6-azauridine, carmofur, cytarabine, doxifluridine, emitefur, enocitabine, floxuridine, fluorouracil, gemcitabine, tagafur), are also useful.

Additional drugs of interest include hydrocortisone, gentamycin, 5-fluorouracil, sorbinil, IL-2, TNF, Phakan-a (a component of glutathione), thiolathiopronin, Bendazac, acetylsalicylic acid, trifluorothymidine, interferon (α, β and γ), immune modulators, e.g., lymphokines, monokines, and growth factors, cytokines, anti-(growth factors), etc. Other pharmacologic agents which may find use maybe found, e.g., in U.S. Pat. Nos. 4,327,725, 4,474,451, and 4,997,652, which disclosures are incorporated herein by reference.

In order to maximize homogeneity of the implants or microimplants, it is desirable that the active ingredient or ingredients be provided in dry form and prepared to uniform particle size. In particular, it is advangtageous, but not required, that the active ingredient or ingredients be provided at a particle size of about 80 µm in diameter or less. Depending on the desired active ingredient, particles can be sorted according to particle size to provide the desired uniform particles. As further detailed herein, such sorting methods can include the use of seives and/or ultracentripetal milling devices. The particles of the active ingredient(s) may be provided within a certain range of sizes. For example, a population of active ingredient particles may have a mean diameter of about 50 µm. Implants formed using such a population may comprise particles within one or two standard deviations from that mean particle diameter. In certain embodiments, the active ingredient particles have a diameter, or other similar size, greater than 3 µm. By controlling the particle size of a population of particles provided in an implant, enhancements in release properties of the active ingredients and therapeutic results can be obtained. For example, implants formed from active ingredient particles having diameters greater than 3 µm provide sustained release of the active ingredient to provide therapeutic benefits that may not be achieved with particles having diameters less than 3 µm.

### Polymers

The selection of the polymeric composition to be employed will vary with the site of administration, the desired period of treatment, patient tolerance, the nature of the disease to be treated and the like. Characteristics of the polymers will include biodegradability at the site of implantation, compatibility with the agent of interest, a desired half-life in a physiological environment, water solubity and the like. The selected polymer or polymer mixtures will usually be anywhere from about 10% and to up to 90% by weight of the formed implant, although other ratios are also contemplated depending on the particular active ingredient and polymer combination and desired release kinetics.

Biodegradable polymeric compositions which may be employed may be organic esters or ethers, which when degraded result in physiologically acceptable degradation products, including the monomers. Anhydrides, amides, orthoesters or the like, by themselves or in combination with other monomers, may find use. The polymers will be condensation polymers. The polymers may be cross-linked or non-cross-linked, usually not more than lightly cross-linked, generally less than 5%, usually less than 1%. For the most part, besides carbon and hydrogen, the polymers will include oxygen and nitrogen, particularly oxygen. The oxygen may be present as oxy, *e.g*., hydroxy or ether, carbonyl, *e.g*., non-oxo-carbonyl, such as carboxylic acid ester, and the like, The nitrogen may be present as amide, cyano and amino. Biodegrable polymers such as those set forth in Heller, Biodegrable Polymers in Controlled Drug Delivery, in: CRC Critical Reviews in Therapeutic Drug Carrier Systems, Viol. 1. CRC Press, Boca Raton, FL (1987), may be used.

Of particular interest are polymers of hydroxyaliphatic carboxylic acids, either homo- or copolymers, and polysaccharides. Included among the polyesters of interest are polymers of D-lactic acid, L-lactic acid, racemic lactic acid, glycolic acid, polycaprolactone, and combinations thereof. By employing the L-lactate or D-lactate, a slowly biodegrading polymer is achieved, while degradation is substantially enhanced with the racemate. Copolymers of glycolic and lactic acid are of particular interest, where the rate of biodegradation is controlled by the ratio of glycolic to lactic acid. The % of polylactic acid in the polylactic acid polyglycolic acid (PLGA) copolymer can be 0-100%, preferably about 15-85%, more preferably about 35-65%. In a particularly preferred embodiment, a 50/50 PLGA copolymer is used. The most rapidly degraded copolymer has roughly equal amounts of glycolic and lactic acid, where either homopolymer is more resistant to degradation. The ratio of glycolic acid to lactic acid will also affect the brittleness of in the implant; a more flexible implant may be desirable depending on the size, shape and intended implantation site of the implant. Also of interest, either alone or in combination, are PLGA copolymers with free acid end groups. The size of the polymer particles is preferably about 1-100 µm in diameter, more preferably about 5-50 µm in diameter, more preferably about 9-12 µm in diameter, still more preferably about 10 µm in diameter. The polymer particles used in the manufacture of the present implants may be provided in a range of sizes. For example, the polymer particles may have a diameter, or other size, from about 5 µm to about 15 µm. In certain embodiments, more than about 50% of the polymer particles in a population of polymer particles have a diameter from about 5 µm to about 15 µm. In additional embodiments, greater than 90% of the polymer particles have a diameter less than 20 µm.

### Additional components

Additionally, release modulators, including accelarators and retardants such as those described in U.S. Patent No. 5,869,079, incorporated herein by reference, may be included in the implants. The amount of release modulator employed will be dependent on the desired release profile, the activity of the modulator, and on the release profile of the active agent in the absence of modulator.

Accelerators may be physiologically inert, water soluble polymers, e.g. low molecular weight methyl cellulose or hydroxypropyl methyl cellulose (HPMC); sugars, e.g. monosaccharides such as fructose and glucose, disaccharides such as lactose, sucrose, or polysaccharides such as cellulose, amylose, dextran, etc. Alternatively, the accelerator may be a physiologically active agent, allowing for a combined therapeutic formulation. The choice of accelerator in such a case will be determined by the desired combination of therapeutic activities.

Release retardants are hydrophobic compounds which slow the rate of release of hydrophilic drugs, allowing for a more extended release profile. Hydrophilic drugs of interest which may benefit from release modulation include water soluble antibiotics, as described above, nucleotide analogs, e.g. acyclovir, gancyclovir, vidarabine, azidothymidine, dideoxyinosine, dideoxycytosine; epinephrine; isoflurphate; adriamycin; bleomycin; mitomycin; ara-C; actinomycin D; scopolamine; and the like.

Agents of interest as release retardants include non-water soluble polymers, e.g. high molecular weight methylcellulose and ethylcellulose, etc., low water soluble organic compounds, and pharmaceutically active hydrophobic agents, as previously described.

Other agents may be employed in the formulation for a variety of purposes. For example, buffering agents and preservatives may be employed. Water soluble preservatives which may be employed include sodium bisulfite, sodium bisulfate, sodium thiosulfate, benzalkonium chloride, chlorobutanol, thimerosal, phenylmercuric acetate, phenylmercuric nitrate, methylparaben, polyvinyl alcohol and phenylethyl alcohol. These agents may be present in individual amounts of from about 0,001 to about 5% by weight and preferably about 0.01 to about 2%. Suitable water soluble buffering agents that may be employed are sodium carbonate, sodium borate, sodium phosphate, sodium acetate, sodium bicarbonate, etc., as approved by the FDA for the desired route of administration. These agents may be present in amounts sufficient to maintain a pH of the system of between 2 to 9 and preferably 4 to 8. As such the buffering agent may be as much as 5% on a weight to weight basis of the total composition. Electrolytes such as sodium chloride and potassium chloride may also be included in the formulation. Where the buffering agent or enhancer is hydrophilic, it may also act as a release accelerator. Hydrophilic additives act to increase the release rates through faster dissolution of the material surrounding the drug particles, which increases the surface area of the drug exposed, thereby increasing the rate of drug bioerosion. Similarly, a hydrophobic buffering agent or enhancer dissolve more slowly, slowing the exposure of drug particles, and thereby slowing the rate of drug bioerosion.

The proportions of active ingredient, polymer, and any other modifiers may be empirically determined by formulating several implants with varying proportions. A USP approved method for dissolution or release test can be used to measure the rate of release (USP 23; NF 18 (1995) pp. 1790-1798). For example, using the infinite sink method, a weighed sample of the drug delivery device is added to a measured volume of a solution containing 0.9% NaCl in water, where the solution volume will be such that the drug concentration is after release is less than 5% of saturation. The mixture is maintained at 37°C and stirred slowly to maintain the implants in suspension. The appearance of the dissolved drug as a function of time may be followed by various methods known in the art, such as spectrophotometrically, HPLC, mass spectroscopy, etc. until the absorbance becomes constant or until greater than 90% of the drug has been released.

### 2. Characteristics of Microimplants

As mentioned, implants and microimplants prepared according to the present methods are highly uniform in size, shape and mass. For microimplants, a high degree of uniformity is especially important because with the small size of the microimplants, especially microimplants having large surface area to volume characteristics, small variations in the microimplant can drastically effect the amount and timing of released drug. In particular, even relatively small localized spots or areas within the microimplant that have greater or lesser concentrations of active ingredient to polymer (so-called "hot spots" or "cold spots") can have deleterious effects. Specifically, any variation in the homogeneity of the mixture, even local variations, can result in the delivery of a drastically different amount of active ingredient than expected, and can alter the treatment of diseases or conditions of the eye. On the other hand, similar localized "hot spots" or "cold spots" in larger implants can still be well tolerated and still provide for an acceptable overall release profile, simply because of the greater overall mass and surface area of the larger implants. Distribution of the active ingredient in the implant can be monitored by examining the content uniformity of a sample of implants of a batch of implants, as understood by persons of ordinary skill in the art.

The present microimplants are preferably monolithic, i.e. having the active ingredient homogenously distributed through the polymeric matrix. Implants which may be produced from a composition that is heated preferably initially include a homogeneous distribution of uniformly-sized particles of active ingredient and uniformly-sized particles of bioerodible polymer. For example, the active ingredient particles and/or polymer particles may be provided in a certain range of sizes, as described above. By using uniformly sized particles, proper mixing of the active ingredient and polymer(s) can be obtained prior to extrusion or further processing of the implant. After or during extrusion, the polymer particles are melted to form a continuous matrix. The selection of the particular polymeric composition to be employed will vary with the desired release kinetics, desired implantation site, patient tolerance, the nature of the disease to be treated and the like. Characteristics of the polymers will include biodegradability at the site of implantation, compatibility with the agent of interest, ease of encapsulation, water insolubility, and the like. Preferably, the polymeric matrix will not be fully degraded until the drug load has been released.

The release kinetics of the microimplants of the invention are dependent in part on their surface area. Larger surface area exposes more polymer to the local environment of the implantation site, causing faster erosion and dissolution of the drug particles entrapped by the polymer. The particular size and shape of the microimplant can be used to control the rate of release, period of treatment, and drug concentration at the site of implantation. Larger implants will deliver a proportionately larger dose, but depending on the surface to volume ratio, may have a slower release rate. The microimplants may take on any size or shape compatible with the selected site of insertion, as long as the microimplants have the desired release kinetics. Preferably, the microimplants are formulated from an extruded filament (or by an injection molded filament), which is then cut to produce the desired product. The upper limit for the microimplant size will be determined by factors such as the desired release kinetics, toleration for the implant, size limitations on insertion, ease of handling, etc. The microimplants will also preferably be at least somewhat flexible (i.e. non-brittle) so as to facilitate both insertion of the implant and accommodation of the implant. Microimplants that are too brittle risk breaking apart during implantation and handling. Implants which are brittle may be implanted and/or provided in implant applicators useful for inserting the implant into an eye. As discussed above, when the applicator contains one or more microimplant, the applicator can be inserted and removed from the eye without requiring sutures. Thus, as described herein, the present implants, including flexible or brittle implants, may be understood to be substantially solid elements or drug delivery devices, at least before placement in an eye of a patient. It is further desirable that that the formed microimplants have lengths (sizes) that do not interfere with the patient's vision after being inserted into the eye. Accordingly, the desired length of a microimplant can depend upon the location of insertion. For example, a microimplant that is inserted into the vitreous chamber at the back of the eye can be chosen with a length that is up to about 10 millimeters, and preferably up to 7 millimeters, so as to avoid interfering with the patient's vision. Accordingly, microimplants according to present invention can generally have lengths less than about 10 millimeters for various applications and implantation at various locations within the eye. In the case of transcleral implantation, thickness rather than length is more important.

In an exemplary embodiment of the present implants, microimplants are formed using processes described herein having a cylindrical or otherwise circular cross-sectional area with a diameter of 0.018 inches or less. A microimplant of such dimensions is capable of insertion into the eye through a cannula having dimensions that correspond to a 22 gauge thin wall or extra thin wall needles, which typically have inner diameters of 0.019 to 0.023 inches. In another exemplary embodiment of the present invention, microimplants are formed with a diameter of 0.015 inches or less and which can be passed through a cannula having dimensions corresponding a 23 gauge thin wall or extra thin wall needles having typical inner diameters of 0.016 to 0.020 inches. Cannulas with these dimensions can be inserted and withdrawn from the eye using techniques, methods which are routine to persons of ordinary skill in the art. The cannulas may be a component of implant injecting apparatus and are structured to insert the present implants without causing the eye to leak excessive fluid, despite normal fluid pressures within the eye. Such techniques, methods, and apparatus, allow for self-sealing methods of delivering the microimplant into the eye and obviate the need for suturing or other surgical procedures to repair the puncture site when the cannula is withdrawn.

In other embodiments of the present implants, microimplants having non-circular geometries can be provided according to processes described herein that can be delivered through cannulas having corresponding cross-sectional geometries. For example, in one embodiment of the present implants, such microimplants can be configured to have a cross-sectional area of 0.00025 square inches or more, depending on the particular cross-sectional geometry.

### 3. Manufacturing processes

Microimplants of the dimensions described can be produced according to the manufacturing processes herein with a high degree of uniformity. As a result, batch quantities of microimplants can be prepared where each microimplant is produced within very narrow tolerances for variations in composition, including localized variations, and/or dimensions. In exemplary production methods, microimplants are produced by obtaining particles of one or more active ingredients, polymers, and/or other optional excipients (e.g., release modulators, buffers, etc.) having particle sizes with a rigorously high degree of homogeneity, blending these particles into a mixture, extruding this mixture into filaments, and precisely cutting these filaments into desired microimplants. Further quality control methods are provided for inspecting these microimplants for desired characteristics. Alternately, these highly homogenous filaments can be pelletized and re-extruded or injection molded. In at least one embodiment, the present microimplants are formed by a method which comprises a dual extrusion of the implant material.

More particularly, in accordance with exemplary processes according to the present invention, active ingredient particles, bioerodible polymer particles, and optional excipient particles can be be subjected to sorting methods to achieve particles with highly uniform particle size, and so that when well mixed, the mixture includes a highly uniform dispersement of active ingredient and polymer particles. For example, the particles of the mixture may be within one or two standard deviations of a mean particle size of the mixture of particles. As previously mentioned, if particle size is not well controlled, and there is a relatively wide range of particle sizes of the active ingredient, the larger or smaller particles can contribute to a higher or lower concentrations of the active ingredient in certain localized regions of the mixture and the resulting implants. These regions of higher concentration can lead to inconsistent dosage levels in the bioerodible implants produced from the mixture, and can therefore lead to ineffective treatment of the ocular disease or condition that the bioerodible implant was designed to treat. As also previously mentioned, a certain degree of variations in particle size may be tolerable for larger implants, where in many cases localized variations in active ingredient can essentially be self-cancelling given the overall size of the implant. For microimplants according to the present invention, however, especially ones with a high surface area to volume ratio, such degree of variation can render the microimplant less suitable, as such localized variations may constitute a relatively larger portion of the entire microimplant, and lead to undesirable spikes and or troughs in the desired release kinetics.

Particles can be sorted according to particle size with devices such as a sieve or an ultracentripetal milling device, for example. In an exemplary process, an ultracentripetal milling device is used. The target size of active ingredient, polymer, and optional excipient particles can vary depending upon the final formulation requirements of the individual microimplant. Generally for the polymer, the size of the polymer particles can be between about 1-100 µm, or between about 5-50 µm, or between about 9-12 µm, or about 10 µm. As discussed herein, a major portion of a population of polymer particles may have a diameter less than 20 µm. For example, greater than 90% of a population of polymer particles may have a diameter of less than 20 µm. In certain embodiments, the polymer particles may have a mean particle size of about 10 µm to about 15 µm. Active ingredient particles can have diameters less than about 80 microns, or from about 1µm to about 20µm, or from about 2µm to about 8µm, or about 5µm. As discussed herein, in certain embodiments, the active ingredient particles have a diameter greater than 3µm. In order to achieve optimum results, it may be desirable for the particles to be sorted to tolerances in particle size between +/- 10% of the desired target diameter, preferably +/- 5%, more preferably +/- 2% of the desired target diameter. By providing controlled particle sizes, such as by reducing and/or eliminating randomly occuring fines of the active ingredient or polymers and by reducing and/or eliminating excessively large particles, enhanced uniformity or homogeneity of the active ingredient can be obtained in the present implants.

In one specific embodiment, dexamethasone particles are used to form a microimplant. In this embodiment, all of the dexamethasone particles have a diameter less than 15 µm, and 90% of the dexamethasone particles have a diameter less than or equal to 5µm.

In another specific embodiment, 75% or more of the dexamethasone particles have a diameter less than 10µm, and 99% or more of the dexamethasone particles have a diameter less than 20µm.

After uniform particles meeting the necessary tolerances are obtained, the desired quantities of active ingredient or ingredients are thoroughly blended with a desired quantity of bioerodible polymer particles and optional excipient particles, to create a relatively uniform dispersement of the active ingredient throughout the mixture. The particular relative quantities can be adjusted depending on a variety of factors, including desired release kinetics, etc. Without being so limited, examples of particular proportions of active ingredients and bioerodible polymers that can be used are described in U.S. Patent No. 5,869,079, and U.S. Patent Application No.09/693,008, both of which are incorporated in their entirety herein by reference. One exemplary method for blending the particles includes placing one or more ball bearings, or the like, into a vessel along with a desired quantities of particles. This vessel can then be placed in a commercially available blender, or similar device, and allowed to agitate for a sufficient period of time until the mixture is thoroughly blended.

After the particles are thoroughly blended, they are prepared for extrusion. The compounding and mixing effect of the extrusion process, such as processes using a screw extruder, further provides for and ensures even greater homogeneity of active ingredient and polymer, and the concomitant melting of the polymer about the active ingredient particles further reduces chances for local variations to occur in the final microimplant product.

If a piston extruder is used, typically the blended mixture is transferred in portions to an appropriate packing barrel and placed into a pneumatic device to compact and remove air voids from the mixture. When a piston extruder is employed, additional processing steps may be desired to improve the homogeneity of the implant components. After a first portion of the mixture in the packing barrel is adequately compacted, another portion of the mixture can be added to the packing barrel and the process can be repeated for additional cycles, until the packing barrel is fully loaded. After a desired quantity of the mixture is added to the packing barrel, the packing barrel can be loaded into an extruder and extruded to form filaments.

Twin-screw extruders that melt and continuously stir the mixture as the mixture is extruded into filaments are preferred. It is desirable, although not necessary, to employ twin-screw extruders having conical screws and barrels as opposed to conventional screws and barrels. This combination of melting and continuously stirring the mixture maintains and can even improve the degree of homogeneity of the mixture in the extruded filaments, and thereby guarding against the formation of localized variations in active ingredient along the length of the extruded filament. For even higher homogenity, these extruded filaments can be reduced to pellets by conventional cutting or milling and then be either re-extruded or injection molded.

By controlling the turn speed of the extruder screw, the temperature and the pressure of the barrel of the extruder, and/or the die geometry, filaments are extruded having given geometries. The geometry of the extruder die or nozzle, specifically, the diameter, length-to-diameter (l/d) ratio, and dies or nozzle finish, together with the applied extrusion pull rate, determines the final diameter of the extruded filament. The dies or nozzles typically can have l/d ratios from about 1:1 to 10:1, and can further have varying geometries, including cylindrical or non-circular cross-sectional geometries. As an example, to achieve microimplants capable of delivery through a 22 gauge cannula, a twin screw extruder having e.g. a 5cc capacity (close batch) and capable of use in a continuous mode and conical barrels and screws can be employed. The extruder can be operated at temperatures from 90-110 °C with screw speeds from 100-150 rpm,. In certain embodiments, implants can be produced by extruding the mixture at a first temperature, such as 105 °C, in the barrel of the extruder, and at a second temperature, such as 102°C, in the nozzle of the extruder and at a rotation of 120 rpm. This may be understood to be a first extrusion step. The extruded mixture then may undergo a second extrusion at a temperature of 107°C in the barrel, 90°C in the nozzle and rotated at 100 rpm in the screw. The extruder can be fitted with nozzles in the range of 0.4-0.5 mm diameter, with overall length/diameter ratios ranging from about 1-10, for example from 2 to 6. Resultant filaments can have diameters of 0,019 inches (482.6 µm) or less and can be cut to yield microimplants of the same dimension diameter. In certain embodiments of the present implants, the target diameter of the filaments or microimplants is 0.018 inches (i.e., 457.2 µm).

The pull rate on the extruded filament can also impact the finished diameter of the filament. For example, if the extruded filament is pulled from the extruder at rate such that the diameter of the extruded filament is drawn down, the finished diameter of the extruded filament will be less than that of the extrusion die or nozzle. In this manner, for example, an extruded filament of less than 0.0019 inches in diameter can be formed from an extruder fitted with 0.019 inch diameter or greater extrusion die or nozzle.

As a continuous filament emerges from the extrusion die, this filament can be cut into convenient lengths for further processing and handling, typically around 6 inches or so. After the filaments are extruded and cut to working lengths, the filaments can further be precision cut to desired lengths to form the desired microimplants. The formed microimplant lengths can be as small as 1 mm or less or can be as long as 6 or 7 mm or even longer. Limitations on the length of the formed microimplants are not a function of the manufacturing process per se but rather are a function of practical limitations in use of the microimplants. For example, longer microimplants such as 10 mm or more may impact a patient's vision when placed in the vitreous. Also, such long microimplants may be more prone to breakage in routine handling simply due to their length.

After the second extrusion, the ability to precisely cut the extruded filaments to a uniform length becomes important to obtaining implants or microimplants that are able to uniformly deliver the desired dosage of active ingredient, especially considering that the microimplants have a relatively high length to diameter ratio, and thus a proportionally high surface area to volume ratio. That is, variations in length will have a greater impact on the amount of active agent delivered than would occur if these ratios were smaller, as is the case for typical larger implants. One method for precision cutting the microimplants uses a system that combines an oscillating wire saw and cutting platform or support designed to retain the extruded filament or filaments. In this example, the platform or other support can be configured to hold the filament or filaments in place, e.g., using vacuum pressure. The wire saw is preferably configured with a diamond-embedded wire for greater cutting precision. Relatively smooth cuts in the filaments can be achieved by slowly cutting through the filaments with an oscillating motion. To maintain a smooth precise cut, it is advantageous to remove any debris created during cutting to keep the cut site free of build-up. Again this can be accomplished, e.g., through the use of a vacuum source. After the first desired cut is made, either the platform or the wire saw can be repositioned to allow another cut in the filaments. Alternatively, both the platform and the wire saw can be repositioned to allow another cut in the filament or filaments. This process can be repeated for to produce batch quantities of the desired microimplants.

Another method for producing the present microimplants may comprise using a guillotine cutter device. Preferably, the operation of the cutter device is automated. For example, an automated guillotine cutter device may be used to precisely and reproducibly form implants from the filaments. In certain embodiments, this may be accomplished by moving one or more filaments past a cutting implement which is configured to cut or form implants at a predetermined rate which may be based on the rate at which the filaments are moving. Other methods may be used using the automated guillotine cutter device and the like.

After the filaments are precision cut to the desired lengths to yield a batch of microimplants, each of the microimplants in the batch can be inspected and sorted. A variety of techniques may be employed, each of which will measure specific aspects of the microimplant, e.g., dimensions, mass, weight, content uniformity active ingredient potency, active ingredient release profile, and the like to determine if the microimplant itself and/or the batch of microimplant meets the tolerance requirements for variance. Based on such measurements, a microimplant or batch of microimplants can be accepted for use or rejected.

In one such method, optical measurements can be taken of each microimplant of a batch or population of microimplants from different defined positions or angles. The optical measurements can be compared to an algorithm to determine whether the microimplant includes the desired target amount of active ingredient based on the measured size and shape of the microimplant, and whether the microimplant includes the appropriate shape and/or surface characteristics to effect the appropriate release kinetics of the active ingredient. This comparison may be based on a desired content uniformity, e.g., active ingredient/polymer ratio. Based on this comparison, the microimplant is either accepted or rejected.

In an another method, where the microimplants are a monolithic mixture of active ingredient and bioerodible polymer, acceptance or rejection of the microimplants can be determined based on whether the mass of each of the microimplants is within an allowable tolerance. This tolerance can be determined based on the acceptable dosage levels of the active ingredient. For most applications, tolerances will be +/- 10% by weight of the desired target mass for the implant, preferably +/- 5% by weight, more preferably +/-2 % by weight. Alternatively, since any variations in mass are likely to be normally distributed and thus the mean or average mass will correspond to the target mass, acceptance of a batch of microimplants can be a function of the relative mass standard deviation of the batch, under the principle that the smaller the standard deviation, the smaller the deviation of any one implant from the desired target mass. Again, for most applications, the acceptable mass standard deviation of the batch of microimplants is 6 or less, preferably 4 or less, and more preferably 2 or less. An advantage of using the mass standard deviation criteria is that it can be based on mass measurements of a statistically significant number of microimplants to reliably accept or reject a batch containing a large number of microimplants, rather than individually weighing each and every microimplant of the batch. The tolerance and inspection of the microimplants can be used to examine drug content, content uniformity, and drug release profiles, among other things, either individually or in combination.

### 4. Administration of Implants/Microimplants

As previously mentioned, implants and microimplants according to the invention can be inserted or implanted into various locations of the eye to treat ocular diseases and conditions. Thus, the present implants can be administered to a patient, such as a person, to treat one or more symptoms of an ocular disease, or to completely treat the disease. The implants or microimplants may be implanted at various sites, depending on the shape and formulation of the implant, the condition being treated, etc. Suitable sites include the anterior chamber, posterior chamber, vitreous cavity, suprachoroidal space, subconjunctiva, episcleral, intracorneal, epicorneal and sclera. Suitable sites extrinsic to the vitreous comprise the suprachoroidal space, the pars plana and the like. Implantation may be intrachoroidal or suprachoroidal. The suprachoroid is a potential space lying between the inner scleral wall and the apposing choroid. Implants that are introduced into the suprachoroid may deliver drugs to the choroid and to the anatomically apposed retina, depending upon the diffusion of the drug from the implant, the concentration of drug comprised in the implant and the like. Implantation may also be intraretinal or subretinal, as well as intrascleral or episcleral. Additionally, implantation into the meningeal spaces, the optic nerve, and/or the intraoptic nerve allows for drug delivery into the central nervous system, and provide a mechanism whereby the blood-brain barrier may be crossed.

Implants that are directly introduced into the vitreous can releasse drug in the vitreous cavity allowing the drug ot reach the back of the eye tissue, such as the retina, or to move to the front of the eye to treat a tissue such as the lens or an anterior chamber tissue. Implants and microimplants may be introduced over or into an avascular region. The avascular region may be naturally occurring, such as the pars plana, or a region made to be avascular by surgical methods. Surgically-induced avascular regions may be produced in an eye by methods known in the art such as laser ablation, photocoagulation, cryotherapy, heat oagulation, cauterization and the like. It may be particularly desirable to produce such an avascular region over or near the desired site of treatment, particularly where the desired site of treatment is distant from the pars plana or placement of the implant at the pars plana is not possible. Introduction of implants over an avascular region will allow for diffusion of the drug from the implant and into the inner eye and avoids diffusion of the drug into the bloodstream. Other sites of implantation include the delivery of anti-tumor drugs to neoplastic lesions, e.g. tumor, or lesion area, e.g. surrounding tissues, or in those situations where the tumor mass has been removed, tissue adjacent to the previously removed tumor and/or into the cavity remaining after removal of the tumor.

The implants and microimplants may be administered in a variety of conventional ways, including surgical means, such as surgical incision and placement methods, or through the use of a trocar. As previously indicated, such methods are typically performed in a surgical setting, and may require an incision to be first made in the eye which must be stitched closed upon completion of the procedure. One of the many advantages of microimplants produced according to the present invention is that due to their extremely small size, they can be implanted using self-sealing methods that avoid the incisions and stitching necessitated by such other methods. In particular, self-sealing methods of administering the microimplants can be performed according to techniques, methods known to persons skilled in the art.

### EXAMPLES

The following examples are intended to provide a more thorough understanding of the present invention. It should be recognized, however, that these examples are provided by way of illustration and not by way of limitation.

### Example 1: Preparation of Single-Extruded Microimplants

Microimplants for insertion into the vitreous cavity of the eye were prepared as follows. The active agent dexamethasone was mixed with 50/50 polylactic acid-polyglycolic acid (PLGA) as the biodegradable polymer. The agent and polymer were thoroughly mixed at a ratio of 60/30/10 by weight of dexamethasone (Pharmacia Corp., Peapack, NJ), 50/50 PLGA having a free acid end (RG502H, Boehringer Ingelheim GmbH, Germany), and 50/50 PLGA (RG502, Boehringer Ingelheim GmbH, Germany), respectively.

Batches of 20 g or less of the drug-polymer blend were then fed into a 5cc capacity (close batch) twin screw extruder capable of use in a continuous mode (DACA MicroCompounder, DACA Instruments, Goleta, CA or Haake Minilab, Thermo Haake, Madison, WI). The extruder is configured with conical screws and barrels, as opposed to conventional cylindrical barrels. The extruder was operated at a temperatures of 90-110 °C at screw speeds of 20-30 rpm, 500-2000N. Various nozzles were fitted to the extruder having diameters in the range of 0.4-0.5 mm and an overall length/diameter ratio of 2 to 6. Extruded filaments were collected, typically at lengths of up to 10 inches, with final diameter of the filaments adjusted by the extrusion pull rate.

Filaments of about 0.018 inches in diameter were further precision cut using an automatic guillotine cutter to produce batches of cylindrical microimplants about 0.018 inches in diameter, 6 mm in length, and containing about 700 µg dexamethasone per microimplant.

Similarly, filaments of 0.015 inches in diameter were precision cut to 10 mm lengths, to produce batches of cylindrical microimplants 0.015 inches in diameter, 10 mm in length, and likewise containing about 700 *µ*g dexamethasone per microimplant.

### Example 2: Reparation of Dual-Extruded Microimplants

Batches of 80g of the drug/polymer blend described in Example 1 were formed into extruded filaments using two extrusions with the Haake Minilab. Microimplants were formed using an automatic guillotine cutter, as described in Example 1.

### Example 3: Inspection and Acceptance of Dexamethasone Microimplants (700 µg) Based on Mass Measurements

Batches of microimplants were produced as in Example 2. Portions of the batches were subjected to examination of weight, dimensions, content uniformity, and release profiles. The target mass for each microimplant was about 1.2 mg total (e.g., 1.167 mg), of which 700 µg is dexamethasone and the remainder polymer. Microimplants weighing within +/- 10 % of the target mass (+/- 0.1167 mg) were deemed acceptable (e.g., implants having a weight range from 1.050 mg to 1.284 mg).

Content uniformity of the implants was from 85.0% LS to 115.0% LS. The following table summarizes the results of the weight of implants from one batch of implants.

| Sample No. | Weight (mg) |
|---|---|
| 1 | 1.175 |
| 2 | 1.190 |
| 3 | 1.184 |
| 4 | 1.176 |
| 5 | 1.161 |
| 6 | 1.181 |
| 7 | 1.190 |
| 8 | 1.162 |
| 9 | 1.183 |
| 10 | 1.169 |
| 11 | 1.165 |
| 12 | 1.174 |
| 13 | 1.181 |
| 14 | 1.155 |
| 15 | 1.188 |
| 16 | 1.188 |
| 17 | 1.183 |
| 18 | 1.162 |
| 19 | 1.191 |
| 20 | 1.163 |
| 21 | 1.164 |
| 22 | 1.199 |
| 23 | 1.182 |
| 24 | 1.192 |
| 25 | 1.170 |
| 26 | 1.186 |
| 27 | 1.164 |
| 28 | 1.165 |
| 29 | 1.168 |
| 30 | 1.163 |
| 31 | 1.178 |
| 32 | 1.150 |
| 33 | 1.176 |
| 34 | 1.199 |
| 35 | 1.170 |
| 36 | 1.166 |
| 37 | 1.176 |
| 38 | 1.173 |
| 39 | 1.169 |
| 40 | 1.183 |
| 41 | 1.202 |

The average weight of the above implants was 1.176 mg. This batch of implants had a standard deviation of 0.012 and a % RSD (relative standard deviation) of 1.06%.

In a population of microimplants produced by the method described above, the average diameter of 1321 microimplants was 0.0181 inches with a standard deviation of 0.0002 and a % RSD of 1.10%.

In a population of microimplants produced by the method described above, the average length of 1302 micro implants was 0.2295 inches with a standard deviation of 0.0011 and a % RSD of 0.48%.

In another population of 10 microimplants, the average weight of the microimplants was 1177.8 *µ*g with a standard deviation of 13.77 and a % RSD of 1.17%. The average weight in mg of dexamethasone in this population of microimplants was 0.70 mg with a standard deviation of 0.01 and a % RSD of 1.24. The average percentage of dexamethasone (weight normalized) was 99.11% with a standard deviation of 0.22 and a % RSD of 0.22. The average percentage of dexamethasone with a target weight of 0.7 mg, was 100.06% with a standard deviation of 1.24 and a % RSD of 1.24. The release profile of a portion of this population of microimplants indicated that about 10% of the dexamethasone was released about 7 days after placement in an aqueous environment, about 40-50% of the dexamethasone was released about 14 days after placement in an aqueous environment, and about 70-80% of the dexamethasone was released about 21 days after placement in an aqueous environment.

In another population of 10 microimplants, the average weight of the microimplants was 1160.5 *µ*g with a standard deviation of 8.33 and a % RSD of 0.72%. The average weight in mg of dexamethasone in this population of microimplants was 0.684 mg. The average percentage of dexamethasone (weight normalized) was 98.14% with a standard deviation of 0.50 and a % RSD of 0.51. The average percentage of dexamethasone with a target weight of 0.7 mg, was 97.62% with a standard deviation of 0.75 and a % RSD of 0.77. The release profile of a portion of this population of microimplants indicated that less than 10% of the dexamethasone was released about 7 days after placement in an aqueous environment, about 50-70% of the dexamethasone was released about 14 days after placement in an aqueous environment, and about 80-90% of the dexamethasone was released about 21 days after placement in an aqueous environment.

In another population of 10 microimplants, the average weight of the microimplants was 1.1582 mg with a standard deviation of 0.02 and a % RSD of 2.1%. The average weight in mg of dexamethasone in this population of microimplants was 0.682 mg. The average percentage of dexamethasone (weight normalized) was 98.02% with a standard deviation of 0.24 and a % RSD of 0.25. The average percentage of dexamethasone with a target weight of 0.7 mg, was 97.31% with a standard deviation of 2.18 and a % RSD of 2.24. The release profile of a portion of this population of microimplants indicated that less than 10% of the dexamethasone was released about 7 days after placement in an aqueous environment, about 40-60% of the dexamethasone was released about 14 days after placement in an aqueous environment, and about 70-80% of the dexamethasone was released about 21 days after placement in an aqueous environment.

### Conclusion

Although the present invention has been described with respect to certain embodiments, configurations, examples, applications, and experiments, it will be apparent to those skilled in the art that various modifications and changes may be made without departing from the invention.

## Claims

1. An ocular microimplant for implantation in the eye comprising:
a homogeneous mixture of one or more active ingredients and one or more bioerodible polymers, wherein said microimplant has a diameter of 0.019 inches or less, 0.015 inches or less, 10 millimeters or less, 7 millimeters or less, or preferably 1 millimeter or less.

2. The microimplant of claim 1, wherein said one or more bioerodible polymers is polylactic acid polyglycolic acid (PLGA) copolymer.

3. The microimplant of claim 1 or 2, wherein said active ingredient is an anti-inflammatory agent, wherein preferably said anti-inflammatory agent is a steroidal anti-inflammatory agent selected from the group consisting of cortisone, dexamethasone, hydrocortisone, methylprednisolone, prednisolone, prednisone, and triamcinolone.

4. The microimplant of 3, wherein said anti-inflammatory agent is dexamethasone and said microimplant further comprises hydroxypropylmethylcellulose (HPMC).

5. A batch of ocular micro implants comprising:
a plurality of microimplants according to claim 1, having a desired target mass per microimplant, and each microimplant has a mass within 10% by weight and preferably a mass within 5% by weight of the desired target mass.

6. The batch of microimplants according to claim 5, wherein each microimplant has a diameter of 0.015 inches or 381 microns or less.

7. A batch of ocular microimplants according to claim 5, wherein the mass standard deviation of the plurality of microimplants is 5 or less, and preferably 2 or less.
